# EUROPÄISCHE PATENTSCHRIFT

(11) **EP 2 150 738 B1**
(45) Veröffentlichungstag und Bekanntmachung des Hinweises auf die Patenterteilung: **13.10.2010**
(21) Anmeldenummer: 08734429.7
(22) Anmeldetag: 22.03.2008
(51) Int. Cl.: F16L 5/08

(54) **ANORDNUNG ZUM ABZWEIGEN EINER FLÜSSIGKEIT AUS EINEM ROHR ODER ZUM ZUFÜHREN EINER FLÜSSIGKEIT IN EIN ROHR**
ASSEMBLY FOR DIVERTING A LIQUID FROM A PIPE OR FEEDING A LIQUID INTO A PIPE
ENSEMBLE PERMETTANT DE DETOURNER UN LIQUIDE D'UN TUBE OU D'AMENER UN LIQUIDE DANS UN TUBE

(30) Priorität: 18.04.2007 DE 102007018595
(43) Veröffentlichungstag der Anmeldung: 10.02.2010
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen
(86) Internationale Anmeldenummer: PCT/DE2008/000499
(87) Internationale Veröffentlichungsnummer: WO 2008/128497

(56) Entgegenhaltungen:
- AU-B2- 536 301
- US-A- 3 944 264
- US-A- 4 621 839

## Beschreibung

Die Erfindung betrifft eine Anordnung zum Abzweigen einer Flüssigkeit aus einem Rohr oder zum Zuführen einer Flüssigkeit in ein Rohr, wobei das Rohr mit einem seitlich wegführenden Rohrstutzen verbunden ist, der an seinem freien Ende einen Flansch aufweist und in den eine Schnellkupplung eingeschraubt ist, wobei der Rohrstutzen an einem Montagerahmen befestigt ist, durch den die Schnellkupplung hindurch verläuft.

Derartige Rohr- und Schlauchinstallationen werden beispielsweise an Dialysestationen verwendet, um einem Dialysegerät die notwendigen extrakorporalen Flüssigkeiten zuzuführen und die von dem Dialysegerät zurückströmende, verbrauchte Flüssigkeit über einen Abwasseranschluss abzuführen. Die Abzweigstellen von den Rohren bzw. Zuführstellen zu den Rohren können dabei an einer Mediensäule vorgesehen sein, wie sie in der DE 195 28 160 C2 offenbart, die zur Befestigung der Anordnung aus Rohrstutzen und Schnellkupplung leistenförmige Montagerahmen enthält.

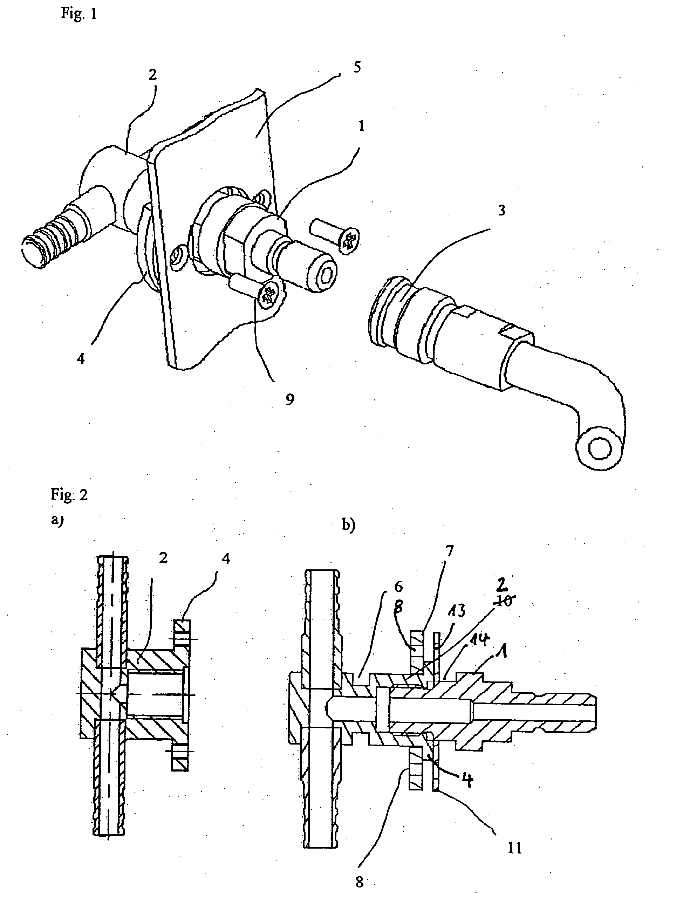

Die Figuren 1 und 2a zeigen eine herkömmliche Anordnung der betrachteten Art, bei der ein Rohrstutzen 2 fest an einem Rohr angebracht ist und durch das Rohr fließende Flüssigkeit im rechten Winkel zur Längsachse des Rohres ableiten kann. Der Rohrstutzen 2 ist an seinem freien Ende mit einem Flansch 4 versehen, der zwei Gewindebohrungen enthält. Ein Montagerahmen 5 liegt an dem Flansch 4 an und enthält zwei vorgefertigte Löcher, durch die Schrauben führen, die in die Gewindebohrungen des Flansches 4 eingeschraubt werden, um den Flansch 4 und damit den Rohrstutzen 2 an dem Montagerahmen 5 zu befestigen. In den Rohrstutzen 2 wird eine Schnellkupplung 1, die mit einem Gewinde versehen ist, eingeschraubt.

Diese Ausbildung hat mehrere Nachteile. Auf die Schnellkupplung 1 wird eine Verbindungsleitung 3 aufgesetzt, die beim Konnektieren/Diskonnektieren im Laufe der Zeit den Gewindesitz der Schnellkupplung derart lockert, dass es hier zu Leckagen kommen kann. Ein weiterer Nachteil besteht darin, dass das Rohr nicht in einer einzigen, festgelegten Richtung, z.B. horizontal verlaufen kann, sondern auch im Winkel zur Horizontalen, woraus sich Probleme dabei ergeben, den Flansch 4 mit seinen vorgefertigten Gewindebohrungen an dem Montagerahmen 5 mit seinen vorgefertigten Löchern anzuschrauben. Die unflexible Montagemöglichkeit ist bei variablen Verlauf des Rohres nur mit einem beträchtlichen Arbeitsaufwand anzupassen.

Der vorliegenden Erfindung liegt die Aufgabe zugrunde, eine Anordnung der betrachteten Art so zu verbessern, dass die Forderung nach Leckagefreiheit und Wirtschaftlichkeit bei der Montage besser als beim Stand der Technik erfüllt ist.

Diese Aufgabe wird erfindungsgemäß durch die Merkmale des Patentanspruchs 1 gelöst.

Vorteilhafte Ausgestaltung der Erfindung sind in den Unteransprüchen gekennzeichnet.

Die Erfindung sieht vor, dass ein Klemmelement auf dem Rohrstutzen angeordnet ist und an dem Flansch anliegt, dass ein Verdrehsicherungselement auf der Schnellkupplung angeordnet ist und an der anderen Seite des Flansches anliegt, dass die Montagewand an der anderen, von dem Flansch abgewandten Seite des Verdrehsicherungselementes anliegt und dass das Klemmelement mit dem Montagerahmen verspannt ist.

Das Verdrehsicherungselement stellt dabei sicher, dass die in den Rohrstutzen eingeschraubte Schnellkupplung sich nicht drehen kann, wenn die auf die Schnellkupplung aufgesetzte Verbindungsleitung konnektiert oder abgenommen wird, so dass ausgeschlossen ist, dass sich der Gewindesitz der Schnellkupplung in dem Rohrstutzen lockert. Der Flansch des Rohrstutzens und das Verdrehsicherungselement sind zwischen dem auf dem Rohrstutzen sitzenden Klemmelement und dem Montagerahmen fest eingespannt, so dass der Rohrstutzen in jeder Winkellage an dem Montagerahmen befestigbar ist. Der Flansch des Rohrstutzens benötigt keine Gewindebohrungen, da der Flansch nicht angeschraubt, sondern fest geklemmt wird.

Das Klemmelement ist vorteilhafterweise ein Klemmring, der vor dem Verspannen frei drehbar auf dem Rohrstutzen sitzt. Weiter ist vorteilhafterweise vorgesehen, dass in der Außenseite des Rohrstutzens eine Ringnut ausgebildet ist und dass der Klemmring ein offener Ring ist mit einer solchen nach außen führenden Öffnung, dass er an der Nut auf den Rohrstutzen aufsetzbar ist. Die mittige kreisbogenförmige Öffnung des Klemmrings hat einen Durchmesser, der im wesentlichen dem Außendurchmesser des kreiszylindrischen Rohrstutzens zwischen Nut und Flansch entspricht.

Der Klemmring hat zweckmäßigerweise wenigstens zwei Gewindebohrungen zum Eingriff von Schrauben, mit denen der Klemmring in bevorzugter Ausgestaltung der Erfindung in Richtung des Montagerahmens angezogen wird. Dabei hat der Flansch des Rohrstutzens einen Rand, der radial innen bezüglich der Gewindebohrungen des Klemmrings liegt, so dass die Schrauben - bevorzugt zwei diametral gegenüberliegende Schrauben - freien Zugang zu den Gewindebohrungen haben.

Das Verdrehsicherungselement hat in vorteilhafter Ausgestaltung der Erfindung eine mittige Aussparung mit einer Kontur, die formschlüssig auf der Schnellkupplung sitzt. Die Schnellkupplung, die zweckmäßigerweise mit einem Maulschlüssel oder dergleichen in dem Rohrstutzen festgeschraubt wird, hat hierzu zwei einander gegenüberliegende ebene Flächen, die durch kreiszylindrische Abschnitte verbunden sind. Das Verdrehsicherungselement hat näherungsweise die Form eines halben Rings mit gegenüberliegenden geradlinigen Wandabschnitten, die durch einen kreisbogenförmigen Randabschnitt verbunden sind, so dass die Schnellkupplung unbeweglich in dem Verdrehsicherungselement gehalten ist, wenn das Verdrehsicherungselement durch eine Klemmkraft in Drehrichtung fixiert ist.

Das Verdrehsicherungselement hat zum Durchgriff der Befestigungsschrauben ein kreisbogenförmiges Langloch, das sich etwa über den gesamten Halbkreis erstreckt. Es können auch z.B. zwei aneinander anschließende kreisbogenförmige Langlöcher vorgesehen sein.

Das Langloch des Verdrehsicherungselementes liegt auf einem solchen Radius, dass die vorzugsweise zwei Löcher in dem Montagerahmen und die Gewindebohrungen des Klemmrings mit dem Langloch des Verdrehsicherungselementes fluchten, so dass die Befestigungsschrauben durch das Langloch hindurchgeführt und in die Gewindebohrungen eingeschraubt werden können.

Durch Festziehen der Befestigungsschrauben werden der Flansch des Rohrstutzens und das Verdrehsicherungselement unbeweglich fixiert. Die Befestigung kann infolge der Drehbarkeit des Klemmrings auf dem Rohrstutzen bei jeder Winkellage des Rohres und damit des Rohrstutzens bewerkstelligt werden.

Weitere Merkmale der Erfindung ergeben sich aus der nachfolgenden Beschreibung einer bevorzugten Ausführungsform der erfindungsgemäßen Anordnung sowie anhand der Zeichnungen. Dabei zeigen:
- Figur 2b): einen Längsschnitt durch die Anordnung ohne Montagerahmen;
- Figur 3: eine Seitenansicht der Anordnung mit Montagerahmen;
- Figur 4: eine perspektivische Ansicht der Anordnung gemäß Figur 3 mit teilweise weggeschnittenem Montagerahmen;
- Figur 5: eine perspektivische Ansicht des bei der Anordnung verwendeten Klemmelementes und
- Figur 6: eine perspektivische Ansicht des bei der Anordnung verwendeten Verdrehsicherungselementes.

Die Figuren 2b), 3 und 4 zeigen, dass auf dem mit einem Rohr verbundenen Rohrstutzen 2 ein Klemmring 7 angeordnet ist, der zwei diametral einander gegenüberliegende Gewindebohrungen 8 aufweist. Der Klemmring 7 liegt an der dem Rohr zugewandten Ringfläche des Flansches 4 des Rohrstutzens 2 an.

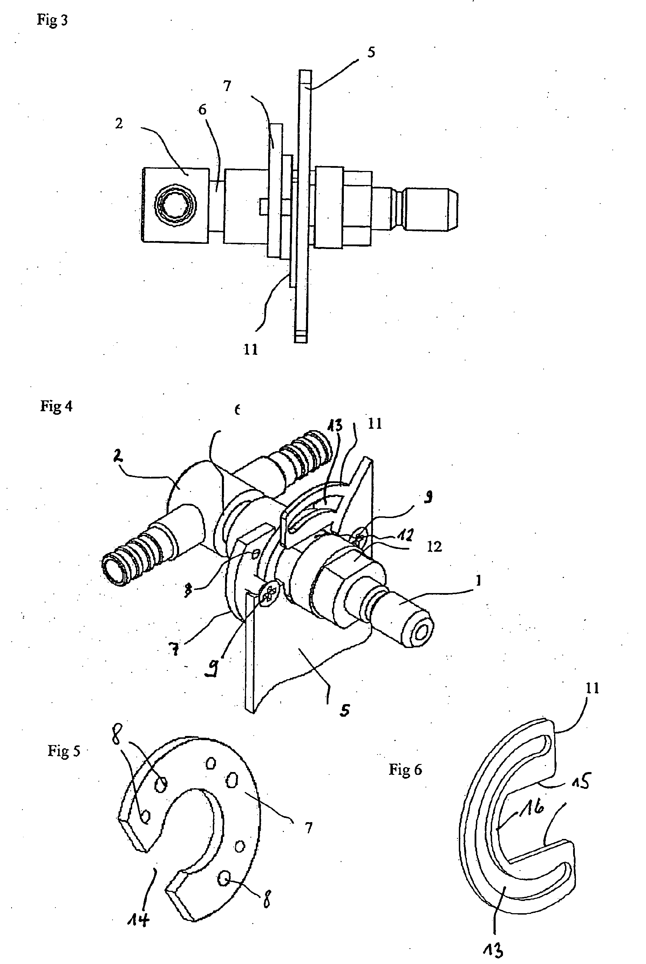

An der gegenüberliegenden Ringfläche des Flansches 4 liegt ein Verdrehsicherungselement 11 an, das formschlüssig einen Umfangsabschnitt der Schnellkupplung 1 umgreift. Das Verdrehsicherungselement 11 hat - wie insbesondere Figur 6 zeigt - ein kreisbogenförmiges Langloch 13, das mit den Gewindebohrungen 8 des Klemmrings 7 fluchtet.

Der Flansch 4 hat einen solchen Durchmesser, dass er radial innerhalb der Gewindebohrungen 8 des Klemmrings 7 und des kreisbogenförmigen Langlochs 13 des Verdrehsicherungselementes 11 endet.

Der Rohrstutzen 2 enthält eine Ringnut 6, die das Aufsetzen des Klemmrings 7 auf den Rohrstutzen 2 ermöglicht, wie weiter unten in Verbindung mit Figur 5 beschrieben wird.

Ein Vergleich der Figuren 2a) und 2b) zeigt, dass der Rohrstutzen 2 der erfindungsgemäßen Anordnung gegenüber der herkömmlichen Ausführung einen kleineren Flansch hat und einen geringeren Materialeinsatz erfordert.

Die Figuren 3 und 4 zeigen, dass an der von dem Rohr abgewandten Seite des Verdrehsicherungselementes 11 ein Montagerahmen 5 anliegt, an der die Anordnung aus Rohrstutzen 2 und Kupplungselement 1 befestigt wird. Hierzu werden zwei Befestigungsschrauben 9 durch Löcher in der Montagewand 5 geführt und in die Gewindebohrungen 8 des Klemmrings 7 eingeschraubt, wobei sie das Langloch 13 des Verdrehsicherungselementes durchgreifen.

Figur 5 zeigt, dass der Klemmring 7 ein offener Ring ist mit einer Eintrittsöffnung 14, die so breit ist, dass der Ring an der Nut 6 auf den Rohrstutzen 2 aufgesetzt werden kann. Der Klemmring 7 wird dann in Anlage an den Flansch 4 verschoben.

Das Verdrehsicherungselement 11 hat ungefähr die Form eines Halbrings mit einer Innenkontur, die mit der Außenkontur des Kupplungselementes 1 an dessen Abschnitt 14 so übereinstimmt, dass das Verdrehsicherungselement 11 auf diesen Abschnitt 14 seitlich aufgeschoben werden kann. Dabei legen sich einander gegenüberliegende geradlinige Randabschnitt 15 an ebene Schlüsselflächen 12 des Kupplungselementes 1 an, während ein kreisbogenförmiger Randabschnitt 16 an einem entsprechenden zylindrischen Abschnitt des Kupplungselementes 1 anliegt.

Wenn die Befestigungsschrauben 9 festgezogen werden, werden der Flansch 4 des Rohrstutzens 2 und das Verdrehsicherungselement 11 fest zwischen dem Klemmring 7 und dem Montagerahmen 5 eingeklemmt, wodurch das Kupplungselement 1 gegen jegliche Drehung gegenüber dem Rohrstutzen 2 fixiert ist.

Figur 5 lässt erkennen, dass das Klemmelement 7 mit mehreren über den Umfang verteilten Gewindebohrungen 8 versehen sein kann.

Die Erfindung ist nicht auf die beschriebene Ausführungsform beschränkt, sondern es liegen zahlreiche Modifikationen und Änderungen im Rahmen des Erfindungsgedankens. Insbesondere können alle offenbarten Merkmale nicht nur auf die beschriebene Weise miteinander kombiniert werden, sondern die Merkmale sind einzeln auf jede denkbare Weise miteinander kombinierbar.

## Patentansprüche

1. Anordnung zum Abzweigen einer Flüssigkeit aus einem Rohr oder zum Zuführen einer Flüssigkeit in ein Rohr, wobei das Rohr mit einem seitlich wegführenden Rohrstutzen (2) verbunden ist, der an seinem freien Ende einen Flansch (4) aufweist und in den eine Schnellkupplung (1) eingeschraubt ist, wobei der Rohrstutzen an einem Montagerahmen (5) befestigt ist, durch den die Schnellkupplung hindurch verläuft,
**dadurch gekennzeichnet,**
**dass** ein Klemmelement (7) auf dem Rohrstutzen (2) an dem Flansch (4) anliegt, dass ein Verdrehsicherungselement (11) auf der Schnellkupplung (1) an der anderen Seite des Flansches (4) anliegt, dass die Montagewand (5) an der anderen Seite des Verdrehsicherungselementes (11) anliegt und dass das Klemmelement (7) mit dem Montagerahmen (5) verspannt ist, wodurch der Flansch (4) und das Verdrehsicherungselement (11) fest eingespannt sind.

2. Anordnung nach Anspruch 1,
**dadurch gekennzeichnet,**
**dass** das Klemmelement (7) ein Klemmring ist, der vor dem Verspannen frei drehbar auf dem Rohrstutzen (2) sitzt.

3. Anordnung nach Anspruch 2,
**dadurch gekennzeichnet,**
**dass** in der Außenseite des Rohrstutzens (2) eine Ringnut (6) ausgebildet ist und dass der Klemmring (7) ein offener Ring ist, derart, dass er an der Nut (6) auf den Rohrstutzen (2) aufsetzbar ist.

4. Anordnung nach einem der Ansprüche 2 oder 3,
**dadurch gekennzeichnet,**
**dass** der Klemmring (7) wenigstens zwei Gewindebohrungen (8) aufweist.

5. Anordnung nach einem der Ansprüche 1 bis 4,
**dadurch gekennzeichnet,**
**dass** das Verdrehsicherungselement (11) eine mittige Aussparung mit einer Kontur aufweist, die formschlüssig auf der Schnellkupplung (1) sitzt.

6. Anordnung nach Anspruch 5,
**dadurch gekennzeichnet,**
**dass** das Verdrehsicherungselement (11) wenigstens ein kreisbogenförmiges Langloch (13) aufweist.

7. Anordnung nach einem der Ansprüche 1 bis 6,
**dadurch gekennzeichnet,**
**dass** der Montagerahmen Löcher für Schrauben (9) aufweist, die das wenigstens eine Langloch (13) des Verdrehsicherungselementes (11) durchgreifen und in die Gewindebohrungen (8) des Klemmrings (7) eingeschraubt sind.

## Claims

1. An assembly for diverting a liquid from a pipe or feeding a liquid into a pipe, the pipe being connected to a laterally extending pipe connection (2) which at its free end comprises a flange (4) and has a quick-action coupling (1) screwed thereinto, the pipe connection being fastened to a mounting frame (5) through which the quick-action coupling extends,
**characterized in**
**that** a clamping element (7) rests against the flange (4) on the pipe connection (2), that an anti-rotation element (11) rests against the other side of the flange (4) on the quick-action coupling (1), that the mounting wall (5) rests against the other side of the anti-rotation element (11), and that the clamping element (7) is fixed to the mounting frame (11), whereby the flange (4) and the anti-rotation element (11) are fixedly clamped.

2. The assembly according to claim 1,
**characterized in**
**that** the clamping element (7) is a clamping ring which prior to fixing is freely rotatably seated on the pipe connection (2).

3. The assembly according to claim 2,
**characterized in**
**that** an annular groove (6) is formed in the outside of the pipe connection (2), and that the clamping ring (7) is an open ring configured such that it is mountable at the groove (6) on the pipe connection (2).

4. The assembly according to any one of claims 2 or 3,
**characterized in**
**that** the clamping ring (7) comprises at least two threaded holes (8).

5. The assembly according to any one of claims 1 to 4,
**characterized in**
**that** the anti-rotation element (11) comprises a central recess with a contour seated on the quick-action coupling (1) in form-fit fashion.

6. The assembly according to claim 5,
**characterized in**
**that** the anti-rotation element (11) comprises at least one elongated hole (13) in the form of a circular arc.

7. The assembly according to any one of claims 1 to 6,
**characterized in**
**that** the mounting frame comprises holes for screws (9) that pass through the at least one elongated hole (13) of the anti-rotation element (11) and are screwed into the threaded holes (8) of the clamping ring (7).

## Revendications

1. Ensemble permettant de détourner un liquide d'un tube ou d'amener un liquide dans un tube, le tube étant relié à une tubulure (2) s'éloignant latéralement qui présente sur son extrémité libre une bride (4) et dans laquelle est vissé un raccord rapide (1), la tubulure étant fixée sur un cadre de montage (5), au travers duquel s'étend le raccord rapide,
**caractérisé en ce**
**qu'**un élément de serrage (7) sur la tubulure (2) repose sur la bride (4), en ce qu'un élément de blocage de rotation (11) sur le raccord rapide (1) repose sur l'autre côté de la bride (4), en ce que la paroi de montage (5) repose sur l'autre côté de l'élément de blocage de rotation (11) et en ce que l'élément de serrage (7) est tendu avec le cadre de montage (5), ce qui permet de serrer fixement la bride (4) et l'élément de blocage de rotation (11).

2. Ensemble selon la revendication 1,
**caractérisé en ce**
**que** l'élément de serrage (7) est un anneau de serrage qui loge de manière à pouvoir tourner librement sur la tubulure (2) avant la tension.

3. Ensemble selon la revendication 2,
**caractérisé en ce**
**que** dans le côté extérieur de la tubulure (2) est réalisée une rainure annulaire (6) et en ce que l'anneau de serrage (7) est un anneau ouvert de telle manière qu'il puisse être placé sur la rainure (6) sur la tubulure (2).

4. Ensemble selon l'une quelconque des revendications 2 ou 3,
**caractérisé en ce**
**que** l'anneau de serrage (7) présente au moins deux trous taraudés (8).

5. Ensemble selon l'une quelconque des revendications 1 à 4,
**caractérisé en ce**
**que** l'élément de blocage de rotation (11) présente un évidement médian avec un contour qui loge par complémentarité de formes sur le raccord rapide (1).

6. Ensemble selon la revendication 5,
**caractérisé en ce**
**que** l'élément de blocage de rotation (11) présente au moins un trou oblong en forme d'arc de cercle (13).

7. Ensemble selon l'une quelconque des revendications 1 à 6,
**caractérisé en ce**
**que** le cadre de montage présente des trous pour des vis (9) qui pénètrent dans l'au moins un trou oblong (13) de l'élément de blocage de rotation (11) et sont vissées dans les trous taraudés (8) de l'anneau de serrage (7).
